# EUROPÄISCHE PATENTSCHRIFT

(11) **EP 1 867 073 B1**
(45) Veröffentlichungstag und Bekanntmachung des Hinweises auf die Patenterteilung: **22.05.2013**
(21) Anmeldenummer: 05822878.4
(22) Anmeldetag: 18.11.2005
(51) Int. Cl.: H04B 10/114, A61B 6/00

(54) **MEHRKANAL-DATENÜBERTRAGUNGSSYSTEM FÜR COMPUTERTOMOGRAPHEN**
MULTI-CHANNEL DATA TRANSFER SYSTEM FOR COMPUTER TOMOGRAPHS
SYSTEME DE TRANSMISSION DE DONNEES MULTICANAL POUR TOMODENSITOMETRES

(30) Priorität: 31.03.2005 DE 102005015034; 14.06.2005 DE 102005027632
(43) Veröffentlichungstag der Anmeldung: 19.12.2007
(73) Patentinhaber: Schleifring und Apparatebau GmbH, 82256 Fürstenfeldbruck (DE)
(72) Erfinder: KRUMME, Nils, 82340 Feldafing (DE)
(74) Vertreter: Lohr, Georg
(86) Internationale Anmeldenummer: PCT/EP2005/012375
(87) Internationale Veröffentlichungsnummer: WO 2006/102935

(56) Entgegenhaltungen:
- DE-A1- 3 530 939
- US-A- 4 646 086

## Beschreibung

### Technisches Gebiet

Die Erfindung betrifft ein Datenübertragungssystem zur Übertragung von Daten zwischen dem rotierenden Teil und dem stationären Teil eines Computertomographen sowie einen Computertomographen mit einem entsprechenden Übertragungssystem.

### Stand der Technik

Aus der US 6,433,631 B1 ist eine Vorrichtung zur Datenübertragung in Computertomographen bekannt. Eine Streifenleitung im rotierenden Teil wird mit dem Sendesignal beaufschlagt. Am stationären Teil ist ein Abgriff vorgesehen, welcher in einem geringen Abstand in einer Größenordnung von ca. 1 mm von der Streifenleitung geführt wird. Mit dieser Vorrichtung kann nur ein Datenkanal (Datenstrom) gleichzeitig übertragen werden. Weiterhin können Signalstörungen oder Signalverluste auftreten, wenn der Abgriff gerade die Trennstelle der Leitung mit den Abschlusswiderständen passiert. Besonders kritisch ist diese Situation bei unterschiedlichen Leitungslängen, wie diese beispielsweise aufgrund von Fertigungstoleranzen auftreten können.

In der US 6,327,327 B1 ist eine Vorrichtung mit mehreren Sendern und mehreren Empfängern zur gleichzeitigen Übertragung mehrerer Signale offenbart. Problematisch ist hier jedoch die Umschaltung zwischen verschiedenen Schleifkontakten bzw. Leitern, wenn der Schleifkontakt gerade ein Leitersegment verlässt und auf das nächste Leitersegment wechselt. Sobald die Laufzeiten des Signals durch ein Leitersegment in der Größenordnung eines Datenbits liegen, wird das Signal verzerrt, bzw. es werden Bits ausgelassen, oder auch mehrfach übertragen.

In der US 6,650,843 ist eine optische Übertragungsvorrichtung mit mehreren Sendern und mehreren Empfängern offenbart, die ebenfalls das zuvor geschilderte Problem aufweist.

In der DE 35 30 939 A1 ist ein optischer Drehübertrager für Computertomographen nach dem Noniusprinzip offenbart. Es ist hier von einer Vielzahl von Sendern und einer Vielzahl von Empfängern positionsabhängig jeweils nur eine Sender/Empfängerkombination im Eingriff.

In der US 4,646,086 ist ein optischer Drehübertrager für Computertomographen offenbart, bei dem zwei Sender Signale in zwei Empfänger einspeisen, wobei die Signale gegenläufig um den Umfang des Drehübertragers übertragen werden.

### Darstellung der Erfindung

Aufgabe der Erfindung ist es, ein Datenübertragungssystem mit wahlweise mehreren Sendern und/oder mehreren Empfängern vorzustellen, bei dem an den Trennstellen zwischen Sendern und/oder Empfängern keine Datenverluste beziehungsweise Übertragungsstörungen auftreten.

Eine erfindungsgemäße Lösung dieser Aufgabe ist in den unabhängigen Patentansprüchen angegeben. Weiterbildungen der Erfindung sind Gegenstand der abhängigen Ansprüche.

Eine erfindungsgemäße Vorrichtung zur Übertragung von Daten zwischen dem rotierenden Teil 1 und dem stationären Teil 2 eines Computertomographen umfasst wenigstens eine Datenquelle 4 am rotierenden Teil und wenigstens eine Datensenke 5 am stationären Teil. Eine Datenquelle kann beispielsweise ein Röntgendetektor 103 bzw. dessen DAS (Data Acquisition System), oder auch beliebige andere Steuereinrichtungen oder Rechner sein. Eine Datensenke kann ein Rechner 106 zur Auswertung und Aufbereitung der Daten, aber auch eine andere Steuereinheit sein.

Weiterhin ist im rotierenden Teil wenigstens eine erste Sendeeinrichtung 8 sowie eine von dieser gespeiste Sendeleiteranordnung 6 vorgesehen. Eine solche erste Sendeeinrichtung empfängt Daten von der Datenquelle und setzt diese zur Übertragung durch die Sendeleiteranordnung um. Die Sendeleiteranordnung umfasst wenigstens einen Leiter zur Führung elektromagnetischer Wellen, der vorzugsweise entlang wenigstens eines Kreissegments oder einer Kreisbahn am rotierenden Teil angebracht ist. Die Sendeleiteranordnung kann beispielsweise mechanische Schleifringe, kontaktlose elektrische Koppelelemente, wie induktive oder kapazitive Koppelelemente, oder aber auch Lichtwellenleiter umfassen. Ebenso kann die Sendeleiteranordnung eine Kombination aus mehreren unterschiedlichen Koppelelementen umfassen.

Weiterhin ist im stationären Teil wenigstens eine erste Empfangseinrichtung 9 vorgesehen, welche von einer Empfangskoppleranordnung 7 gespeist wird. Die Empfangskoppleranordnung weist wenigstens zwei Koppler auf, wobei zu jedem Zeitpunkt der Übertragung wenigstens ein Koppler im Eingriff mit der Sendeleiteranordnung steht.

Die Koppler sind passend zur Sendeleiteranordnung ausgebildet. So können beispielsweise kapazitive Koppelflächen in Verbindung mit einer Streifenleitungsstruktur als Sendeleiteranordnung eingesetzt werden. Ebenso können auch optische Prismenkoppler in Verbindung mit einem Lichtwellenleiter, beispielsweise wie einem Spiegelgraben als Sendeleiteranordnung kombiniert werden.

Die Empfangseinrichtung setzt die von der Empfangskoppleranordnung 7 aus der Sendeleiteranordnung 6 empfangenen Signale zur Weiterleitung an die Datensenke um. Die Empfangseinrichtung umfasst wenigstens zwei Empfänger sowie eine Auswerteeinrichtung zur Auswertung der Signale der Empfänger. Eine Auswerteeinrichtung wertet Signale wenigstens eines Empfängers aus und selektiert die Daten wenigstens eines Empfängers zur Weiterleitung an die Datensenke aufgrund wenigstens eines Qualitätskriteriums. An Stelle der Selektion der Ausgangs-Daten eines Empfängers könnten auch Signale im Inneren eines Empfängers oder auch dessen Eingangsignale selektiert beziehungsweise umgeschaltet werden. Bei einer Vorrichtung zur gleichzeitigen Übertragung mehrerer Signale (mehrerer Kanäle) wird eine der Anzahl der zu übertragenden Signale entsprechende Anzahl von Signalen der Empfänger selektiert. Wird beispielsweise nur ein einziger Kanal übertragen, so wird auch nur ein Signal eines Empfängers selektiert und an die Datensenke weitergeleitet.

Neben einem Qualitätskriterium können auch weitere Umschaltsignale, beispielsweise Positionssignale oder auch im Datenstrom enthaltene Signale zur Umschaltung herangezogen werden.

Mit einer erfindungsgemäße Vorrichtung können wahlweise ein Datenstrom oder auch mehrere Datenströme gleichzeitig übertragen werden. Ebenso kann auch ein Datenstrom in mittels eines Multiplexers in der Datenquelle oder in der Sendeeinrichtung in mehrere parallele Datenströme aufgeteilt werden. Die Daten können in Form von einzelnen Datenpaketen übertragen werden. Vorteilhafterweise wird der Inhalt so kodiert, das Übertragungsfehler innerhalb der Datenpakete erkannt werden können. In einem Demultiplexer in der Empfangseinrichtung oder der Datensenkekönnen die Daten auf korrekte Übertragung geprüft, sowie eventuell doppelt übertragene Datenpakete verworfen oder fehlende Datenpakete erneut angefordert werden.

Die Vorrichtung ist vorteilhafterweise derart ausgebildet, dass eine Signalübertragung in jedem Drehwinkel zwischen Rotor und Stator erfolgen kann. Alternativ kann eine Übertragung nur bei bestimmten Positionen oder in bestimmten Bereichen erfolgen.

Selbstverständlich ist eine erfindungsgemäße Übertragungsvorrichtung nicht nur bei Computertomographen, sondern auch bei anderen medizinischen Diagnostiksystemen und ebenso bei allgemeinen Drehübertragungsaufgaben einsetzbar. Ebenso ist es bei allgemeinen Übertragungsaufgaben und insbesondere bei linear bewegten Teilen anwendbar.

Eine b_{[GL2]} esonders vorteilhafte Ausgestaltung der Erfindung sieht als wenigstens ein Qualitätskriterium wahlweise eine Eingangssignalamplitude, einen Signal/Rauschabstand, eine Jitteramplitude, eine spektrale Verteilung der Signale, eine Frame-Fehlerrate, eine Paritätsfehlerrate und/oder eine Bitfehlerrate vor.

Beispielsweise kann aus mehreren Empfängern derjenige Empfänger ausgewählt werden, welcher die beste Qualität aufweist. Zweckmäßigerweise ist noch eine Hysterese vorgesehen, um zu häufiges Umschalten zu vermeiden.

Die _{[GL3]} Steuerung der Auswahl kann auch durch mehrere Qualitätskriterien gesteuert werden. So kann ein Auswahlkriterium basierend auf einer mit festgelegten oder dynamisch errechneten Faktoren gewichteten Kombination aus mehreren Parametern, beispielsweise mit unterschiedlicher Gewichtung, oder sogar mit zeitlicher _{[GL4]} Abfolge oder zeitabhängiger Gewichtung erfolgen. Beispielsweise könnte eine erste Auswahlentscheidung aufgrund eines schnell zu messenden Parameters, wie einer Signalamplitude getroffen werden. Zur Optimierung könnte dann ein mit größerer Messdauer behaftetes Kriterium, wie eine Bitfehlerrate hinzugezogen werden.

In _{[GL5]} einer weiteren Ausgestaltung der Erfindung sind die für das Übertragungsmedium spezifischen Komponenten, wie Sendeeinrichtung 8, Sendeleiteranordnung 6, Empfangseinrichtung 9 und Empfangskoppleranordnung 7 zur Übertragung optischer Signale ausgelegt.

Alternativ k_{[GL6]} önnen auch die für das Übertragungsmedium spezifischen Komponenten, wie Sendeeinrichtung 8, Sendeleiteranordnung 6, Empfangseinrichtung 9 und Empfangskoppleranordnung 7 zur Übertragung elektrischer Signale oder auch elektromagnetischer Felder und Wellen ausgelegt.

In einer weiteren vorteilhaften Ausgestaltung der Erfindung ist die Sendeleiteranordnung in mehrere Segmente unterteilt und die Empfangskoppleranordnung weist wenigstens einen Koppler mehr als die Anzahl der Segmente der Sendeleiteranordnung auf. Damit ist eine lückenlose Übertragung ohne Datenverluste möglich.

Eine _{[GL8]}andere Ausgestaltung der Erfindung sieht wenigstens einen Pufferspeicher zur Zwischenspeicherung empfangener Daten vor, mithilfe dessen Datenverluste bei Übergängen beziehungsweise Umschaltvorgängen zwischen verschiedenen Empfängern verhindert werden können. Durch einen solchen Zwischenspeicher können fallweise einzelne Bits oder auch größere Datenpakete gespeichert werden.

Die _{[GL9]} weitere Ausgestaltung der Erfindung sieht das Löschen mehrfach empfangener Daten vor.

In _{[GL10]} einer weiteren Ausgestaltung ist die Sendeleiteranordnung (6) in mehrere Segmente unterteilt, wobei die einzelnen Segmente derart von der Sendeeinrichtung (8) mit Signalen gespeist werden, dass wahlweise Bit-Takte oder Frame- Takte benachbarter Segmente nahezu phasengleich sind. Dies hat den Vorteil, dass eine oder mehrere in Empfängern vorhandene PLLs bei Übergängen zwischen Segmenten der Sendeleiteranordnung durch einzelne Koppler nicht aus der Synchronisation fallen. Die Datenverluste einzelner Bits oder Frames können durch einen entsprechenden Pufferspeicher ausgeglichen werden.

In _{[GL11]} einer weiteren Ausgestaltung wird ein Datenstrom niedrigerer Datenrate zum Haupt-Datenstron hinzugemultiplext. Dieser hat vorzugsweise eine Taktrate mit einem ganzzahligen Verhältnis zur Datenrate des Hauptdatenstroms. Der Empfänger kann den Datenstrom durch Oversampling decodieren und anschließend digital korrigieren. Alternativ können auch zwei parallele Empfangskanäle unterschiedlicher Datenrate eingesetzt werden.

Ein erfindungsgemäßer Computertomograph umfasst wenigstens eine der hier angegebenen Vorrichtungen zur Kommunikation.

Zur Vereinfachung der Darstellung wird in diesem Dokument auf eine Übertragung von dem rotierenden Teil auf das feststehende Teil eines Computertomographen Bezug genommen. Selbstverständlich ist eine erfindungsgemäße Vorrichtung auch in umgekehrter Übertragungsrichtung einsetzbar. Ebenso kann eine erfindungsgemäße Vorrichtung auch in anderen Anwendungen zur Drehübertragung und ebenso zur linearen Übertragung zweier gegeneinander beweglichen Einheiten herangezogen werden.

Die Übertragungsrichtung entsprechend dem Anspruch 1 wurde vom Rotor zum Stator gewählt, da dies dem häufigsten Einsatzfall entspricht. Allerdings ist ebenso eine Übertragung in entgegengesetzter Richtung oder aber auch bidirektional möglich.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Fig. 1 zeigt in allgemeiner Form schematisch einen Computertomographen.
Fig. 2 zeigt schematisch die Anordnung von Sende/Empfangseinrichtungen.
Fig. 3 zeigt schematisch eine Anordnung mit optischer Übertragung.
Fig. 4 zeigt schematisch eine elektrische Übertragungseinrichtung.

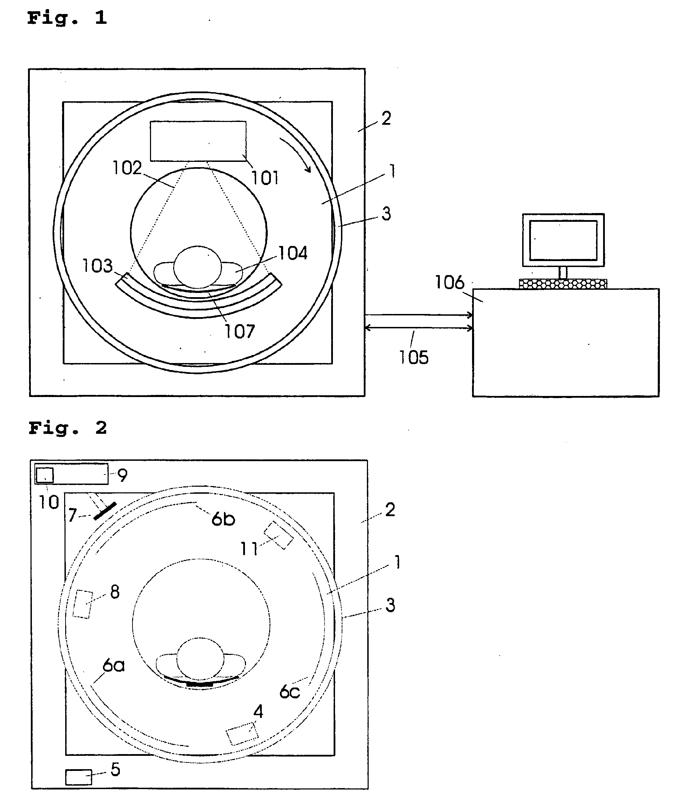

Fig. 1 zeigt beispielhaft eine erfindungsgemäße Vorrichtung. Der Computertomograph (CT-Scanner) besteht aus zwei mechanischen Hauptbestandteilen. Ein stationäres Teil 2 dient als Basis und Träger des ganzen Gerätes, in denen sich das rotierende Teil 1 dreht. Der Patient 104 wird auf einer Liege 107 in der Öffnung des rotierenden Teils positioniert. Zur Abtastung des Patienten mittels Röntgenstrahlen 102 ist eine Röntgenröhre 101 sowie ein dieser gegenüberliegend angeordneter Detektor 103 vorgesehen. Röntgenröhre 101 und Detektor 103 sind auf dem rotierenden Teil 1 drehbar angeordnet. Ein Drehübertrager 3 dient zur elektrischen Verbindung zwischen dem rotierenden Teil 1 und dem stationären Teil 2. Hierbei werden einerseits die hohe elektrische Leistung zur Speisung der Röntgenröhre 101 in Richtung des rotierenden Teils 1 und gleichzeitig die Rohdaten des Bildes in der entgegengesetzten Richtung übertragen. Parallel hierzu ist eine Kommunikation von Steuerinformationen in beiden Richtungen vorgesehen. Eine Auswerte- und Steuereinheit 106 dient zur Bedienung des Computertomographen sowie zur Anzeige der erzeugten Bilder. Die Kommunikation mit dem Computertomographen erfolgt über eine bidirektionale Verbindung 105.

Fig. 2 zeigt in vereinfachte Form eine beispielhafte Anordnung eines erfindungsgemäßen Computertomografen mit zur Übertragung benötigten Komponenten. Die Daten einer Datenquelle 4 (Detektor 103 mit anschließender Signalverarbeitung bzw. DAS) an dem rotierenden Teil 1 werden mittels einer ersten Sendeeinrichtung 8 aufbereitet und an die Sendeleiteranordnung, welche hier beispielhaft aus drei Teilen 6a, 6b, 6c dargestellt ist, weitergeleitet. Diese Sendeleiteranordnung führt nun die hochfrequenten Signale. Diese werden von der Empfangskoppleranordnung 7 abgegriffen. Es ist beispielhaft eine Empfangskoppleranordnung, welche fest mit dem stationären Rahmen verbunden ist, dargestellt. Die von dieser Empfangskoppleranordnung 7 aufgefangenen Signale werden an eine erste Empfangseinrichtung 9 zur Aufbereitung weitergeleitet. Deren Ausgangsignale werden dann zu einer Datensenke 5 geführt.

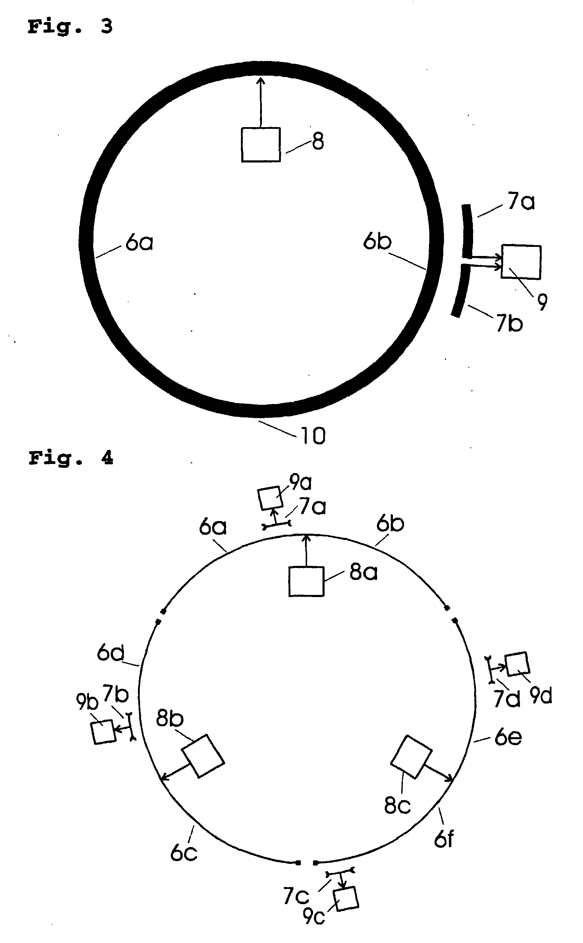

In Figur 3 ist in vereinfachter Form eine Anordnung mit optischer Übertragung dargestellt. Die Sendeeinrichtung 8 ist als optische Sendeeinrichtung ausgebildet und speist optische Signale in eine optische Sendeleiteranordnung 6 bestehend aus einem geschlossenen optischen Sendeleiter. Zur anschaulichen Darstellung wird der idealerweise einstückig ausgebildete Sendeleiter in eine erste Hälfte 6a und eine zweite Hälfte 6b unterteilt. Optische Signale werden am Ort der Sendeeinrichtung 8 eingespeist und breiten sich in beiden Hälften 6a und 6b in entgegengesetzten Richtungen aus. Vorteilhafterweise befindet sich an der Endposition 10 ein optischer Absorber. Eine erfindungsgemäße Anordnung kann aber auch ohne einen solchen Absorber arbeiten. In diesem Falle ist es wünschenswert, eine Sendeleiteranordnung mit signifikanter Dämpfung, vorzugsweise größer 6 dB pro Umdrehung, besonders Vorteilhafterweise größer 12 dB pro Umdrehung einzusetzen. Hier kann also durch Einsatz der Erfindung ein preiswerter Sendeleiter mit höherer Dämpfung verwendet werden. Die Empfangskoppleranordnung weist zwei richtungsselektiven Empfangskoppler 7a und 7b auf. Im vorliegenden Falle sei der Empfangskoppler 7a zum Empfang von Signalen im Uhrzeigersinn und der Empfangskoppler 7b von Signal entgegen dem Uhrzeigersinn ausgelegt. Die Signale dieser Empfangs Koppler werden zur Empfangseinrichtung 9 geführt und dort weiterverarbeitet. Vorteilhafterweise wird das Signal desjenigen Empfangskopplers 7a, 7b ausgewählt, welches die niedrigste Bitfehlerrate oder die niedrigste Frame-Fehlerrate aufweist. Ein besonders günstiger Algorithmus ist die Überwachung auf Frame- bzw. Parity-Fehler und eine Umschaltung auf den anderen Empfangskoppler, sobald bei einem Empfangskoppler eine bestimmte Anzahl von Fehlern aufgetreten ist, und bei dem anderen Empfangskoppler gleichzeitig keine oder weniger Fehler aufgetreten sind.

Die Funktionsweise dieser Anordnung soll an Hand eines Umlaufs der Empfangseinrichtung mit Empfangskopplern entlang der Sendeleiteranordnung, beginnend vom Punkt der Sendeeinrichtung 8 entgegen dem Uhrzeigersinn dargestellt werden. Am Ort der Sendeeinrichtung 8 empfangen beide Empfangskoppler 7a und 7b jeweils gleiche starke Signale, welche sich ausgehend vom Ort der Sendeeinrichtung 8 in Richtung der beiden Hälften 6a und 6b der Sendeleiteranordnung fortpflanzen. Welcher der beiden Empfangskoppler nun zur Ausgabe der Signale selektiert ist, hängt von den Ausgangsbedingungen des vorhergehenden Umlaufes ab. Bewegt sich nun die Empfangseinrichtung entgegen dem Uhrzeigersinn von dem Ort der Sendeeinrichtung weg, so wird der zweite Empfangskoppler 7b das auf kurzem Wege übertragene Signal von der Sendeeinrichtung 8 empfangen, während der erste Empfangskoppler 7a das nahezu entlang des ganzen Umfangs übertragene und daher stark gedämpfte Signal empfängt. Somit wird für die Signalübertragung der zweite Empfangskoppler 7b selektiert. An der Endposition 10 sind die von beiden Empfangskoppler empfangenen Signale gleich stark. Bei weiterer Bewegung und somit wieder zunehmender Annäherung an die Sendeeinrichtung 8 erhält der erste Empfangskoppler 7a das auf dem kürzeren Wege im Uhrzeigersinn übertragene Signal, während der zweite Empfangskoppler 7b das auf dem längeren Wege übertragene und somit stärker gedämpfte Signal erhält. Nun erfolgt eine Selektion des ersten Empfangskopplers 7a. Es ist ersichtlich, dass jeweils ohne eine Steuerung durch zusätzliche Positionssignale eine Umschaltung auf den jeweils günstigeren Empfangskoppler erfolgt.

In Figur 4 ist eine weitere erfindungsgemäße Anordnung mit in drei Abschnitten und jeder Abschnitt jeweils in zwei Segmente unterteilten kapazitiven Sendeleitersegmenten dargestellt. In dem ersten Abschnitt werden die beiden Hälften 6a und 6b des Sendeleiters von der Sendeeinrichtung 8a in deren Mitte gespeist. Im zweiten Abschnitt sind die beiden Hälften 6c und 6d durch die Sendeeinrichtung 8b gespeist. Schließlich sind noch im dritten Abschnitt die beiden Hälften 6e und 6f durch die Sendeeinrichtung 8c gespeist. Die Sendeeinrichtungen 8a, 8b, 8c können auch Bestandteil einer gemeinsamen Sendeeinrichtung sein. Die Einspeisung eines Signals kann auch an einem Ende eines Sendeleiterstückes erfolgen. Die Sendeleiter sind Vorteilhafterweise reflexionfrei abgeschlossen. Sie sind im vorliegenden Beispiel zu Übertragung von drei unterschiedlichen Datenströmen (Kanälen) vorgesehen. Zum Empfang sind die vier Empfangskoppler 7a, 7b, 7c, 7d, jeweils verbunden mit einem Empfänger 9a, 9b, 9c, 9d vorgesehen. Diese sind alle fest miteinander verbunden und bewegen sich gleichzeitig gegenüber den Sendern. Dadurch, dass wenigstens ein Empfänger mehr als die Anzahl der Sender vorgesehen ist, ist eine unterbrechungsfreie Übertragung um den gesamten Umfang möglich, selbst wenn ein Empfangskoppler gerade zwischen zwei Sendeleiterstücken steht, da dann immer noch wenigstens jeweils ein Empfangskoppler mit den benachbarten Sendeleiterstücken im Eingriff ist. Die Umschaltung der Kanalzuordnung kann beispielsweise gesteuert durch die Signalamplitude in einem Empfänger oder auch durch eine Bitfehlerrate erfolgen, da bei demjenigen Empfänger beziehungsweise dessen Empfangskoppler, der gerade den Übergang zwischen zwei Sendeleiter-Segmenten passiert, ein Einbruch der Signalamplitude und ein Anstieg der Bitfehlerrate zu beobachten ist. Zusätzlich beziehungsweise alternativ könnte auch in den Daten eines jeden Kanals eine Kennung des Kanals mit übertragen werden. In der Empfangseinrichtung kann dann durch Auswertung dieser Information wieder eine Zuordnung der Empfänger zu den Kanälen erfolgen.

Die Paketgröße der Datenpakete (Frames) ist optimiert auf Drehzahl, Dauer von Übertragungsstörungen in Folge einer Überlappung der Antennen, Resynchronisierungszeit der PLL und Wellenausbreitungsgeschwindigkeit auf der Sendeantenne, so dass eine Störung bevorzugter Weise maximal die Dauer einer Paketlänge aufweist. Damit sind maximal zwei Pakete gestört.

Die in den Ausführungsbeispielen dargestellten Merkmale sind wahlweise auf optische, elektrische oder andere Übertragungsmedien anwendbar und weiterhin unabhängig von der Anzahl der zu übertragenden Kanäle.

### Bezugszeichenliste

| | |
|---|---|
| 1 | Rotierendes Teil |
| 2 | Stationäres Teil |
| 3 | Drehübertrager |
| 4 | Datenquelle |
| 5 | Datensenke |
| 6 | Sendeleiteranordnung |
| 7 | Empfangskoppleranordnung |
| 8 | Sendeeinrichtung |
| 9 | Empfangseinrichtung |
| 10 | Endposition eines oder mehrerer Senderleiter |
| 101 | Röntgenröhre |
| 102 | Röntgenstrahlung |
| 103 | Detektor |
| 104 | Patient |
| 105 | bidirektionale Verbindung |
| 106 | Auswerte- und Steuereinheit |
| 107 | Patientenliege |

## Patentansprüche

1. Vorrichtung zur Übertragung von Daten zwischen dem rotierenden Teil (1) und dem stationären Teil (2) eines Computertomographen,
wobei eines der Teile (1), (2)
■ wenigstens eine Datenquelle (4), wie einen Röntgendetektor,
■ wenigstens eine Sendeeinrichtung (8) zum Empfang von Daten von der Datenquelle (4) und Aussendung von Signalen,
■ eine von dieser Sendeeinrichtung gespeiste Sendeleiteranordnung (6) zur Führung der Signale entlang wenigstens eines vorgegebenen Bereiches des Rotors, vorzugsweise in Form wenigstens eines Kreissegments oder einer Kreisbahn aufweist und
das jeweils andere Teil (2), (1)
■ eine Empfangskoppleranordnung (7) zum Abgriff von Signalen der Sendeleiteranordnung (6),
■ wenigstens eine Empfangseinrichtung (9) zum Empfang von Signalen der Empfangskoppleranordnung,
■ sowie eine Datensenke zur Auswertung bzw. Weiterverarbeitung der von der Empfangseinrichtung gelieferten Daten umfasst, und
die Empfangskoppleranordnung (7) wenigstens zwei Koppler aufweist, wobei zu jedem Zeitpunkt der Übertragung wenigstens ein Koppler im Eingriff mit der Sendeleiteranordnung (6) steht, die Empfangseinrichtung wenigstens zwei mit jeweils wenigstens einem Koppler verbundene Empfänger sowie eine Auswerteeinrichtung zur Auswertung von Signalen der Empfänger umfasst,
**dadurch gekennzeichnet dass**,
die Auswerteeinrichtung aufgrund wenigstens eines vorgegebenen Qualitätskriteriums aus Frame- Fehlerrate, Paritätsfehlerrate und/oder einer Bitfehlerrate der Signale der Empfänger die Daten wenigstens eines Empfängers zur Weiterleitung an die Datensenke selektiert.

2. Vorrichtung zur Übertragung von Daten nach Anspruch 1
**dadurch gekennzeichnet dass**,
die Auswerteeinrichtung aufgrund wenigstens eines vorgegebenen Qualitätskriteriums der Signale, nämlich eine durch vorgegebene Gewichtungsfaktoren gewichtete Kombination aus mehreren einzelnen Qualitätskriterien wie beispielsweise einer Eingangssignalamplitude, eines Signal/Rauschabstands, einer Jitteramplitude, einer spektralen Verteilung der Signale, einer Frame- Fehlerrate, einer Paritätsfehlerrate oder einer Bitfehlerrate der Empfänger, die Daten wenigstens eines Empfängers zur Weiterleitung an die Datensenke selektiert.

3. Vorrichtung nach Anspruch 1 oder 2,
**dadurch gekennzeichnet dass**,
wenigstens ein Qualitätskriterium durch eine zeitlich variable Abfolge mehrerer einzelner Qualitätskriterien wie beispielsweise einer Eingangssignalamplitude, eines Signal/Rauschabstands, einer Jitteramplitude, einer spektralen Verteilung der Signale, einer Frame- Fehlerrate oder einer Bitfehlerrate gebildet wird.

4. Vorrichtung nach einem der vorhergehenden Ansprüche,
**dadurch gekennzeichnet dass**,
Sendeeinrichtung (8), Sendeleiteranordnung (6), Empfangseinrichtung (9) und Empfangskoppleranordnung (7) zur Übertragung optischer Signale ausgelegt sind.

5. Vorrichtung nach einem der vorhergehenden Ansprüche,
**dadurch gekennzeichnet dass**,
Sendeeinrichtung (8), Sendeleiteranordnung (6), Empfangseinrichtung (9) und Empfangskoppleranordnung (7) zur Übertragung elektrischer Signale ausgelegt sind.

6. Vorrichtung nach einem der vorhergehenden Ansprüche,
**dadurch gekennzeichnet dass**,
die Sendeleiteranordnung (6) in mehrere Segmente unterteilt ist und die Empfangskoppleranordnung (7) wenigstens einen Koppler mehr als die Anzahl der Segmente der Sendeleiteranordnung aufweist.

7. Vorrichtung nach einem der vorhergehenden Ansprüche,
**dadurch gekennzeichnet dass**,
einen Pufferspeicher zur Zwischenspeicherung empfangener Daten vorgesehen ist.

8. Vorrichtung nach einem der vorhergehenden Ansprüche,
**dadurch gekennzeichnet dass**,
mehrfach empfangene Daten verworfen werden.

9. Vorrichtung nach einem der vorhergehenden Ansprüche,
**dadurch gekennzeichnet dass**,
die Sendeleiteranordnung (6) in mehrere Segmente unterteilt ist, wobei die einzelnen Segmente derart von der Sendeeinrichtung (8) mit Signalen gespeist werden, dass wahlweise Bit- Takte oder Frame- Takte benachbarter Segmente nahezu phasengleich sind.

10. Vorrichtung nach einem der vorhergehenden Ansprüche,
**dadurch gekennzeichnet dass**,
im Multiplexverfahren wenigstens ein Datenkanal niedrigerer Datenrate zu dem Datenstrom gemultiplext wird.

11. Computertomograph umfassend eine Vorrichtung nach einem der vorhergehenden Ansprüche.

## Claims

1. Device for transmitting data between the rotating part (1) and the stationary part (2) of a computer tomograph,
wherein one of the parts (1), (2) has
■ at least one data source (4) such as an X-ray detector,
■ at least one transmission means (8) for receiving data from the data source (4) and transmitting signals,
■ a transmission conductor arrangement (6) which is fed by this transmission means for guiding the signals along at least a predetermined region of the rotor, preferably in the form of at least one circular segment or a circular path, and
the respective other parts (2), (1) comprises
■ a receiving coupler arrangement (7) for picking off signals from the transmission conductor arrangement (6),
■ at least one receiving means (9) for receiving signals from the receiving coupler arrangement,
■ and also a data sink for evaluating respectively further processing the data supplied by the receiving means, and
the receiving coupler arrangement (7) has at least two couplers, at least one coupler being engaged with the transmission conductor arrangement (6) at any moment during the transmission, the receiving means comprises at least two receivers, each connected to at least one coupler, and also an evaluation means for evaluating signals from the receivers, **characterized in, that**
the evaluation means selects, on the basis of at least one predetermined quality criterion of frame error rate, parity error rate and/or a bit error rate of the signals from the receivers, the data from at least one receiver for forwarding to the data sink.

2. Device for transmitting data according to claim 1,
**characterised in, that**
the evaluation means selects, on the basis of at least one predetermined quality criterion of the signals, namely a combination, weighted by predetermined weighting factors, of a plurality of individual quality criteria such as, for example, an input signal amplitude, a signal-to-noise ratio, a jitter amplitude, a spectral distribution of the signals, a frame error rate, a parity error rate or a bit error rate of the receivers, the data from at least one receiver for forwarding to the data sink.

3. Device according to claim 1 or 2,
**characterised in, that**
at least one quality criterion is formed by a time-variable sequence of a plurality of individual quality criteria such as, for example, an input signal amplitude, a signal-to-noise ratio, a jitter amplitude, a spectral distribution of the signals, a frame error rate or a bit error rate.

4. Device according to any one of the preceding claims,
**characterised in, that**
the transmission means (8), transmission conductor arrangement (6), receiving means (9) and receiving coupler arrangement (7) are configured to transmit optical signals.

5. Device according to any one of the preceding claims,
**characterised in, that**
the transmission means (8), transmission conductor arrangement (6), receiving means (9) and receiving coupler arrangement (7) are configured to transmit electrical signals.

6. Device according to any one of the preceding claims,
**characterised in, that**
the transmission conductor arrangement (6) is divided into a plurality of segments and the receiving coupler arrangement (7) has at least one coupler more than the number of segments of the transmission conductor arrangement.

7. Device according to any one of the preceding claims,
**characterised in, that**
a buffer memory is provided for the intermediate storage of received data.

8. Device according to any one of the preceding claims,
**characterised in, that**
multiply received data is rejected.

9. Device according to any one of the preceding claims,
**characterised in, that**
the transmission conductor arrangement (6) is divided into a plurality of segments, the individual segments being fed with signals by the transmission means (8) in such a way that bit clock pulses or frame clock pulses of adjacent segments are optionally almost in phase with one another.

10. Device according to any one of the preceding claims,
**characterised in, that**
at least one data the channel having a relatively low data rate is multiplexed to the data stream using the multiplex method.

11. Computer tomograph comprising a device according to any one of the preceding claims.

## Revendications

1. Dispositif pour la transmission de données entre la partie rotative (1) et la partie stationnaire (2) d'un scanographe,
dans lequel l'une des parties (1), (2) comporte
• au moins une source de données (4) telle qu'un détecteur de rayons X,
• au moins un dispositif émetteur (8) pour la réception de données de la source de données (4) et l'émission de signaux,
• une disposition de conducteurs d'émission (6) alimentée par ce dispositif émetteur pour acheminer les signaux le long d'au moins une zone prédéterminée du rotor, de préférence en forme d'au moins un segment de cercle ou d'une trajectoire circulaire,
et l'autre partie (2), (1) comporte
• une disposition de coupleurs de réception (7) pour capter les signaux de la disposition de conducteurs d'émission (6),
• au moins un dispositif récepteur (9) pour recevoir des signaux de la disposition de coupleurs de réception,
• et un collecteur de données pour l'analyse respectivement le traitement des données fournies par le dispositif récepteur, et
la disposition de coupleurs de réception (7) comprend au moins deux coupleurs, au moins un coupleur étant en prise avec la disposition de conducteurs d'émission (6) à tout moment pendant la transmission, le dispositif récepteur comprenant au moins deux récepteurs reliés chacun à au moins un coupleur et un dispositif d'analyse pour l'analyse de signaux du récepteur,
**caractérisé en ce que** le dispositif d'analyse sélectionne les données d'au moins un récepteur à transmettre au collecteur de données en fonction d'au moins un critère de qualité prédéterminé parmi le taux d'erreurs de trame, le taux d'erreurs de parité et/ou un taux d'erreurs de bits.

2. Dispositif pour la transmission de données selon la revendication 1, **caractérisé en ce que** le dispositif d'analyse sélectionne les données d'au moins un récepteur à transmettre au collecteur de données en fonction d'au moins un critère prédéterminé de qualité des signaux, à savoir une combinaison pondérée par des facteurs de pondération prédéterminés entre plusieurs critères de qualité différents tels qu'une amplitude de signal d'entrée, un rapport signal sur bruit, une amplitude de jitter, une répartition spectrale des signaux, un taux d'erreurs de trame, un taux d'erreurs de parité ou un taux d'erreurs de bits du récepteur, par exemple.

3. Dispositif selon l'une des revendications 1 ou 2, **caractérisé en ce qu'**au moins un critère de qualité est constitué par une succession variable dans le temps de plusieurs critères de qualité différents, par exemple une amplitude de signal d'entrée, un rapport signal sur bruit, une amplitude de jitter, une répartition spectrale des signaux, un taux d'erreurs de trame, d'erreurs de parité ou d'erreurs de bits.

4. Dispositif selon l'une des revendications précédentes, **caractérisé en ce que** le dispositif émetteur (8), la disposition de conducteurs d'émission (6), le dispositif récepteur (9) et la disposition de coupleurs de réception (7) sont conçus pour la transmission de signaux optiques.

5. Dispositif selon l'une des revendications précédentes, **caractérisé en ce que** le dispositif émetteur (8), la disposition de conducteurs d'émission (6), le dispositif récepteur (9) et la disposition de coupleurs de réception (7) sont conçus pour la transmission de signaux électriques.

6. Dispositif selon l'une des revendications précédentes, **caractérisé en ce que** la disposition de conducteurs d'émission (6) est divisée en plusieurs segments et la disposition de coupleurs de réception (7) présente au moins un coupleur de plus que le nombre de segments de la disposition de conducteurs d'émission.

7. Dispositif selon l'une des revendications précédentes, **caractérisé en ce qu'**une mémoire tampon est prévue pour l'enregistrement temporaire des données reçues.

8. Dispositif selon l'une des revendications précédentes, **caractérisé en ce que** les données reçues plusieurs fois sont rejetées.

9. Dispositif selon l'une des revendications précédentes, **caractérisé en ce que** la disposition de conducteurs d'émission (6) est divisée en plusieurs segments, les différents segments étant alimentés en signaux par le dispositif émetteur (8) de telle façon que les fréquences de bits ou les fréquences de trames de segments voisins soient presque en phase.

10. Dispositif selon l'une des revendications précédentes, **caractérisé en ce qu'**au moins un canal de données à bas débit de données est multiplexé pour donner le flux de données selon un procédé de multiplexage.

11. Scanographe comprenant un dispositif selon l'une des revendications précédentes.
